# FASCICULE DE BREVET EUROPEEN

(11) **EP 3 241 901 B1**
(45) Date de publication et mention de la délivrance du brevet: **10.07.2019**
(21) Numéro de dépôt: 16168001.2
(22) Date de dépôt: 03.05.2016
(51) Int. Cl.: C12N 15/10, C12M 1/34, C12Q 1/68

(54) **PROCEDE ET SYSTEME EXTRACTION MAGNETIQUE DE COMPOSANTS DANS UN ECHANTILLON LIQUIDE**
MAGNETISCHES EXTRAKTIONSVERFAHREN UND -SYSTEM VON BESTANDTEILEN IN EINER FLÜSSIGEN PROBE
METHOD AND SYSTEM FOR MAGNETIC EXTRACTION OF COMPONENTS IN A LIQUID SAMPLE

(43) Date de publication de la demande: 08.11.2017
(73) Titulaire: bioMérieux, 69280 Marcy l'Etoile (FR)
(72) Inventeur: ALIX, David, 35140 Gosné (FR); MINASSIAN, Edgar, 69210 Lentilly (FR); RAYMOND, Jean-Claude, 69690 Bessenay (FR); WANDELS, Philippe, 69003 Lyon (FR)

(56) Documents cités:
- EP-A1- 1 992 689
- EP-A2- 1 081 234
- WO-A1-03/006168
- WO-A1-2007/051859
- WO-A1-2014/072438
- WO-A1-2014/083165
- US-A- 5 234 809
- US-A1- 2011 076 205

## Description

### DOMAINE DE L'INVENTION

L'invention a trait au domaine de l'extraction de composants contenus dans une solution en utilisant des particules magnétiques.

L'invention trouve particulièrement application dans le domaine de la préparation d'échantillon biologique, notamment dans la mise en oeuvre de diagnostic *in vitro*, par la capture d'analytes d'origine biologique (acides nucléiques, microorganismes, protéines, peptides, etc.) présents dans une solution.

### ETAT DE LA TECHNIQUE

A l'origine développée pour l'extraction d'acides nucléiques présents dans un échantillon biologique et décrite dans le document US 5 234 809, la technologie « BOOM® » consiste à introduire dans un échantillon liquide des particules magnétiques capables de se lier avec des composants d'intérêt, puis à séparer les particules magnétiques de l'échantillon à l'aide d'un ou plusieurs aimants. Les particules ainsi capturées peuvent alors subir un traitement ultérieur par exemple pour libérer leurs composants dans une solution de récupération. En raison de l'efficacité de cette technique, de nombreux dispositifs ont été développés et commercialisés, notamment pour l'ADN et l'ARN, ...), tant des dispositifs manuels (par exemple le NucliSENS-miniMAG® du demandeur) que des dispositifs automatisés (NucliSENS-easyMAG® du demandeur). Ces dispositifs automatisés souffrent cependant de diverses limitations.

Une première limitation concerne leur polyvalence et leur encombrement. En effet, ces dispositifs sont le plus souvent des automates lourds et encombrants qui sont conçus pour mettre en oeuvre une séquence de traitements non modifiable par l'utilisateur. Un automate est ainsi conçu pour un seul type d'extraction, par exemple conçu pour la purification d'acides nucléiques mais incapable de mettre en oeuvre une immuno-concentration magnétique.

Une deuxième limitation concerne les circuits d'injection et d'aspiration des différents liquides utilisés lors de l'extraction. Le nombre de liquides étant important, ceci implique des circuits également nombreux et/ou complexes. De plus, en raison de possibles contaminations, ces circuits d'injection/aspiration doivent être régulièrement nettoyés, ce qui implique la mise hors service des dispositifs.

Une troisième limitation concerne les opérations de brassage qui sont mises en oeuvre pour obtenir l'homogénéité de l'échantillon comprenant les particules magnétiques avant leur capture, pour maximiser la capture par ces dernières des analytes d'intérêt ou pour laver efficacement les particules magnétiques. Ce type de brassage nécessite usuellement des mécanismes complexes, par exemple à base d'aimants mobiles qui mettent en mouvement les particules magnétiques.

La quatrième limitation concerne les différents liquides utilisés lors de l'extraction. Usuellement, les étapes mises en oeuvre pour l'extraction sont réalisées dans un ou à partir d'un seul récipient. De fait, ce récipient fixe un volume identique pour tous les liquides en jeu (e.g. l'échantillon, les différentes solutions de lavage, la solution d'élution, etc...), ce qui limite l'efficacité globale du processus d'extraction. En effet, certains traitements (e.g. le lavage) nécessitent de grands volumes pour être pleinement efficaces alors que d'autres traitements se contentent d'un petit volume de liquide (e.g. l'élution).

### EXPOSE DE L'INVENTION

Le but de la présente invention est de proposer un procédé d'extraction de composants dans un échantillon liquide à l'aide de particules magnétiques qui offre une grande liberté dans le choix des volumes liquides, en particulier jusqu'à 10 ml, utilisés lors de l'extraction.

A cet effet, l'invention a pour objet un procédé d'extraction de composants contenus dans un échantillon biologique sous forme liquide, lesdits composants étant aptes à se fixer sur des particules magnétiques, le procédé comprenant :
- une phase de mélange de l'échantillon avec les particules magnétiques;
- une phase d'aspiration du mélange depuis un puits dans un cône de pipette tubulaire comprenant une pointe destinée au pipetage de liquide;
- une phase de capture des particules magnétiques sur une paroi interne du cône de pipette :
   ∘ en appliquant un premier champ magnétique au cône de pipette, ledit champ étant apte à attirer et maintenir les particules magnétiques dans une zone prédéterminée du cône de pipette, dite de « capture », au-dessus de la pointe de celui-ci ;
   ∘ et en appliquant au moins un cycle d'aspiration et de refoulement du mélange contenu dans le cône de pipette dans un puits;
- au moins une phase de lavage des particules capturées sur la paroi interne du cône de pipette en :
   ∘ refoulant le mélange contenu dans le cône de pipette ; et
   ∘ en appliquant depuis un puits contenant une solution de lavage au moins un cycle d'aspiration et de refoulement de la solution de lavage dans le cône de pipette ;
- une phase de migration des particules magnétiques sur la paroi interne du cône, depuis la zone de capture jusqu'à la pointe du cône de pipette, en réalisant un déplacement relatif du cône de la pipette par rapport au premier champ magnétique ;
- et une phase de transfert desdites particules magnétiques ayant migré dans la pointe du cône de pipette dans un puits de récupération contenant une solution.

En d'autres termes, l'invention tire avantage d'un cône de pipette dans lequel des cycles d'aspiration et de refoulement peuvent être réalisés en trempant sa pointe dans un puits. Grâce à de tels cycles, il est possible de capturer la totalité des particules présentes dans un échantillon de volume très supérieur à celui du volume, i.e. le cône, dans lequel l'extraction est réalisée. Il est même possible de faire passer dans le cône un volume cumulé de liquide bien supérieur au volume de l'échantillon lui-même, en réglant le nombre de cycles d'aspiration et de refoulement. De la même manière, le volume de la ou des solution(s) de lavage utilisée(s) le cas échéant lors de l'extraction peut être très supérieur au volume du cône. La phase de migration permet quant à elle de localiser les particules magnétiques dans une portion du cône, la pointe, qui peut tremper dans un puits de volume très réduit. Le volume de la solution de récupération peut donc être faible si nécessaire. Le volume de la solution de récupération est ainsi indépendant du volume de l'échantillon et du volume du cône de pipette dans lequel est réalisée l'extraction. Grâce à l'invention, il est par conséquent possible d'optimiser chaque volume de liquide utilisé, et ainsi optimiser l'extraction.

De plus, en raison de la géométrie des cônes en forme de tube et des cycles d'aspiration refoulement, il est obtenu un brassage efficace de l'échantillon dans les cônes, brassage par exemple mis en oeuvre avant la capture, ainsi qu'un lavage efficace, et ce sans faire appel à des mécanismes de type aimants mobiles. En outre, les inventeurs ont noté qu'un lavage efficace est obtenu dans le cône alors même que les particules sont capturées sur la paroi du cône de pipette. Un grand volume de solution de lavage peut être utilisé, augmentant encore l'efficacité du lavage. De fait, toutes les étapes de l'extraction (brassage, capture, lavage, transfert dans une solution de récupération) peuvent être réalisées dans le cône de pipette.

Selon un mode de réalisation, le déplacement du premier champ magnétique consiste à déplacer le cône de pipette parallèlement à un axe longitudinal dudit cône, et à conserver constant le premier champ magnétique, l'axe longitudinal du cône de pipette restant à égale distance du premier champ magnétique lors du déplacement du cône de pipette.

En d'autres termes, la migration des particules peut être mise en oeuvre de manière simple en déplaçant le cône de pipette par rapport, par exemple, un aimant permanent.

Selon un mode de réalisation, la phase de transfert comprend :
- le placement de la pointe du cône de pipette dans le puits de récupération ;
- et l'application d'un second champ magnétique depuis le fond du puits de récupération de manière à faire migrer dans le puits de récupération les particules magnétiques contenues dans la pointe du cône de pipette.

En particulier, le second champ magnétique est produit par un aimant positionné partiellement ou entièrement sous la pointe du cône de pipette. Le premier champ magnétique appliqué au cône de pipette est désactivé lors de l'application du second champ magnétique.

En d'autres termes, le second champ magnétique permet d'attirer simplement les particules dans le puits de récupération, ce qui augmente la vitesse de récupération des particules magnétiques dans le puits de récupération, ainsi que le nombre de particules récupérées. De plus, le second champ magnétique capture automatiquement les particules magnétiques dans le puits de récupération. Par exemple, si la solution de récupération est un éluant, les composants liés aux particules ont été libérés et un technicien peut pipeter directement la solution qui est dépourvue de particules magnétiques.

Selon une variante privilégiée, la phase de transfert comporte la désactivation du premier champ magnétique suivie de l'application de cycles d'aspiration et de refoulement de la solution du puits de récupération dans la pointe du cône de pipette, ladite application comprenant :
- une première phase d'application des cycles à une première fréquence ;
- suivie d'une deuxième phase d'application des cycles à une deuxième fréquence, inférieure à la première fréquence.

La première phase permet de désagréger efficacement l'amas de particules capturées sur le cône de pipette, également appelé « culot », et ainsi de remettre en suspension les particules dans la solution de récupération. La deuxième phase permet de continuer à brasser la solution tout en ne s'opposant pas à la migration des particules sous l'effet du champ magnétique. Ceci permet d'augmenter encore plus la vitesse de récupération et le nombre de particules récupérées dans le puits de récupération. De plus si la solution est un éluant, dont la fonction est de libérer les composants capturés par les particules magnétiques, ces cycles ont pour effet de brasser les particules dans l'éluant, ce qui augmente l'efficacité de l'éluant, en particulier lors de l'utilisation d'une solution d'élution dans le décrochage des analytes des particules magnétiques sans étape de chauffage.

Selon un mode de réalisation, le procédé comprend, préalablement à la phase de capture, une phase de brassage du mélange contenu dans le cône de pipette par l'application d'au moins un cycle d'aspiration et de refoulement dudit mélange dans le cône de pipette. En raison de la géométrie du cône, de forme tubulaire, il est possible d'obtenir un grand débit volumique rapporté à la section du cône, et par conséquent un brassage efficace. De plus, il existe de grandes turbulences dans le cône naturellement générées par l'écoulement du liquide, turbulences qui augmentent l'efficacité du brassage. Avantageusement, un accessoire jetable est prévu dans le cône pour accroitre cet effet.

Selon un mode de réalisation, le procédé comprend, préalablement à la phase de transfert, au moins une phase de lavage des particules capturées sur la paroi interne du cône de pipette en :
- en désactivant le champ magnétique ;
- en appliquant depuis un puits contenant une solution de lavage au moins un cycle d'aspiration et de refoulement de la solution de lavage dans le cône de pipette ;
- en appliquant une deuxième phase de capture sur une paroi interne du cône de pipette :
   ∘ en appliquant le premier champ magnétique au cône de pipette
   ∘ et en appliquant au moins un cycle d'aspiration et de refoulement du mélange contenu dans le cône de pipette dans le puits contenant la solution de lavage.

Selon un mode de réalisation, le procédé comprend, préalablement à la phase de transfert, au moins une phase de lavage des particules capturées sur la paroi interne du cône de pipette en :
- en aspirant de la solution de lavage dans ledit cône de pipette ;
- puis en modulant le premier champ magnétique appliqué aux particules magnétiques pour capturer celles-ci sur la paroi interne du cône de pipette ;
- puis en refoulant le liquide de lavage du cône de pipette.

En d'autres termes, la modulation du champ magnétique induit une réorganisation du culot de particules capturé sur la paroi du cône. Notamment, le culot peut changer de forme, s'étaler, glisser ou encore « rouler » sur la paroi du cône de pipette. Cette réorganisation du culot permet d'augmenter encore l'efficacité du lavage, et ce d'autant plus que cette réorganisation peut être mise en oeuvre conjointement avec des cycles d'aspiration et de refoulement de la solution de lavage.

En particulier, la modulation du premier champ magnétique est réalisée :
- en déplaçant le cône de pipette parallèlement à un axe longitudinal dudit cône, et en conservant constant le premier champ magnétique ;
- et/ou en faisant défiler des aimants espacés les uns des autres devant les particules capturées.

En d'autres termes, la modulation est obtenue simplement, par exemple par un technicien qui fait glisser une barrette d'aimants ou par un automate qui met un mécanisme simple de translation d'une barrette d'aimants.

Selon un mode de réalisation, le volume du cône de pipette est au moins dix fois supérieur au volume du puits de récupération. Selon un mode de réalisation, le volume du mélange est au moins trois fois supérieur au volume du cône de pipette.

Selon un mode de réalisation, les composants appartiennent au groupe formé des acides nucléiques simple brin ou double brin (ADN et/ou ARN), des microorganismes, des protéines, et des peptides. Les composants sont constitués de tout autres types de molécules en fonction de la fonctionnalisation données aux particules magnétiques.

Le but de la présente invention est également de proposer un dispositif pour la mise en oeuvre du procédé venant d'être décrit, qui soit peu encombrant et simple d'utilisation par un technicien de laboratoire.

A cet effet, l'invention a également pour objet un porte-pipette comprenant :
- un socle ;
- un évidement formé dans le socle apte à loger de manière amovible un support de puits ;
- un support de pipette comprenant un premier logement dans lequel est apte à être insérée, avantageusement de manière amovible, une pipette équipée d'au moins un cône de pipette tubulaire comprenant une pointe destinée au pipetage de liquide, le premier logement étant ouvert sur l'évidement du socle, le support de pipette étant mobile en translation par rapport au socle selon une direction parallèle à un axe des cônes de pipette et mobile entre une première position dans laquelle la pointe de chaque cône de pipette est logée dans un puits du support de puits et au moins une deuxième position dans laquelle ladite pointe est en dehors dudit puits;
- un second logement apte à loger de manière amovible une pièce aimantée, le second logement faisant face à chacun des cônes de pipette dans une position au-dessus de la pointe de celui-ci lorsque le support de pipette est dans la première position, et le second logement fait face à la pointe du cône de pipette lorsque le support de pipette est dans la deuxième position.

En d'autres termes, le porte-pipette reçoit une pipette et le technicien met en oeuvre les étapes du procédé d'extraction, en montant/descendant la pipette, notamment pour faire migrer le culot de particule dans la pointe du ou des cônes de pipette, en introduisant des puits (sous la forme de plaque, de barrette, etc) dans le socle, et en actionnant la pipette.

Le porte-pipette, qui est peu encombrant et transportable, permet en outre la semi-automatisation du procédé d'extraction lorsqu'une pipette électronique est utilisée. Une telle pipette comprend en effet des circuits d'aspiration et de refoulement dans chaque cône de pipette l'équipant, et un circuit électronique à base de microprocesseur. Ce circuit électronique pilote les circuits d'aspiration/refoulement en fonction de consignes entrées par le technicien au moyen d'une interface équipant la pipette et/ou d'un ordinateur/tablette/smartphone connecté à la pipette (e.g. par une liaison sans fil de type bluetooth), etc. Ces consignes sont par exemple constituées d'instructions de cycles d'aspiration/refoulement et/ou d'un choix d'un protocole particulier préenregistré dans la pipette.

La pipette électronique étant programmable, une grande polyvalence est en outre obtenue dans la définition du procédé d'extraction, qui peut être adapté en fonction d'une capture magnétique particulière souhaitée (e.g. : purification d'acides nucléiques, immuno-concentration magnétiques,...). Notamment, un protocole approprié à l'extraction visée peut être enregistré dans la pipette, le protocole étant défini en termes de nombre de cycles d'aspiration et de refoulement, d'enchaînement de cycles, de fréquence de cycles, de durée entre les cycles, volumes définis, etc. Un système autonome et semi-automatisé est ainsi obtenu.

Enfin, les cônes de pipette sont détachables de la pipette, et donc aisément remplaçables, sans que la pipette ne soit mise hors service pendant une longue durée.

Selon un mode de réalisation, le premier logement comprend une ouverture pour l'insertion et le retrait frontaux de la pipette dans le premier logement du support de pipette. L'insertion et le retrait frontaux de la pipette et des cônes en position minimisent le risque de toucher le porte-pipette avec les pointes des cônes, et donc le risque de contamination du porte-pipette.

Selon un mode de réalisation, le second logement est réalisé dans le socle.

En particulier, le support de pipette comporte un troisième logement dans lequel la pièce aimantée est apte à être logée de manière amovible pour faire face à chaque cône de pipette à une position au-dessus de la pointe dudit cône lorsque le support de pipette est dans la deuxième position.

En d'autres termes, lorsque la pièce aimantée (e.g. comprenant un ou plusieurs aimants permanents) est présente dans le troisième logement, elle est solidaire des cônes de pipette et suit donc leur mouvement de translation par rapport au socle. Lors de tels mouvements, la pièce aimantée conserve donc les culots de particules magnétiques fixes dans les cônes. Le technicien peut ainsi par exemple monter la pipette pour déplacer plus aisément un support à puits dans le socle sans risquer de déplacer les culots de particules dans les cônes.

Selon un mode particulier, le second et troisième logements communiquent, et le support de pipette comprend des moyens aptes à maintenir de manière amovible la pièce aimantée dans le troisième logement. De cette manière, le technicien peut détacher la pièce aimantée du support à pipette qui prend alors place automatiquement dans le socle en tombant dans le deuxième logement. Ce détachement a notamment lieu pour l'opération de migration des particules magnétiques dans les pointes des cônes.

Selon un mode de réalisation :
- le socle comprend au moins une roue dentée mobile en rotation ;
- et le support de pipette comprend une crémaillère coopérant avec la roue dentée pour translater le support de pipette par rapport au socle lors de la rotation de la roue dentée.

En particulier, le porte-pipette comprend un dispositif de verrouillage et de déverrouillage du support de pipette dans la première position. Notamment, le porte-pipette comprend au moins une poignée solidaire de la roue dentée pour faire tourner celle-ci et apte à se fixer de manière amovible à une poignée solidaire de la roue dentée d'un autre porte-pipette, ce qui permet donc d'augmenter le nombre de cônes de pipette pendant le procédé d'extraction.

L'invention a également pour objet un système pour l'extraction de composants contenus dans un échantillon biologique sous forme liquide, lesdits composants étant aptes à se fixer sur des particules magnétiques, le système comprenant :
- une pipette équipée d'au moins un cône de pipette tubulaire comprenant une pointe destinée au pipetage de liquide et d'un circuit d'aspiration et de refoulement dans chaque cône de pipette ;
- au moins un support de puits ;
- un porte-pipette comprenant :
   ∘ un socle ;
   ∘ un évidement formé dans le socle apte à recevoir de manière amovible chaque support de puits ;
   ∘ un support de pipette comprenant un premier logement dans lequel la pipette est insérée, avantageusement de manière amovible, le premier logement étant ouvert sur l'évidement du socle, le support de pipette étant mobile en translation par rapport au socle selon une direction parallèle à un axe des cônes de pipette et mobile entre une première position dans laquelle la pointe de chaque cône de pipette est logée dans un puits du support de puits et au moins une deuxième position dans laquelle ladite pointe est en dehors dudit puits;
   ∘ un second logement faisant face à chacun des cônes de pipette dans une position au-dessus de la pointe de celui-ci lorsque le support de pipette est dans la première position et le second logement faisant face à la pointe du cône de pipette lorsque le support de pipette est dans la deuxième position ; et
- une pièce aimantée logée de manière amovible dans le second logement.

Notamment, le porte-pipette est conforme au porte-pipette décrit plus haut.

L'invention a également pour but de proposer un support de puits pour la migration de particules magnétiques depuis des pointes de cônes de pipettes dans des puits de récupération.

A cet effet, l'invention a également pour objet un support de puits, comprenant une pièce dans laquelle sont formés des évidements pour la réception des puits, et au moins un aimant faisant face à chacun des évidements formés dans ladite pièce.

### BREVE DESCRIPTION DES FIGURES

L'invention sera mieux comprise à la lecture de la description qui va suivre, donnée uniquement à titre d'exemple, et faite en relation avec les dessins annexés, dans lesquels des références identiques désignent des éléments identiques, et dans lesquels :
- la figure 1 est une vue en perspective d'un système d'extraction selon l'invention ;
- les figures 2A et 2B sont des vues de face et en perspective d'une pipette électronique et de ses cônes de pipettes amovibles ;
- les figures 3A et 3B est des vues en perspective et de face d'un porte-pipette selon l'invention ;
- les figures 4A et 4B est une vue de détail en coupe du porte-pipette de la figure 3, respectivement selon les plans A-A et B-B de la figure 3B ;
- la figure 5 est une vue en perspective d'une plaque « deepwell » comprenant des puits ;
- les figures 6A et 6B sont des vues en perspective d'un portoir magnétique et de tubes d'élution PCR pouvant se loger dans le portoir ;
- la figure 7 est une vue de face d'une pièce aimantée selon l'invention ;
- la figure 8 est un organigramme d'un procédé d'extraction selon l'invention ;
- la figure 9 est une photographie des cônes de pipette d'un système selon l'invention avec des culots de particules magnétiques placés environ à mi-hauteur des cônes ;
- la figure 10 est une photographie de ces mêmes cônes avec les culots placés dans les pointes de ceux-ci ;
- la figure 11 est une photographie de tubes d'élution PCR dans lesquels ont été récupérées les particules magnétiques :
- les figures 12 et 13 illustrent un second mode de réalisation du porte-pipette selon l'invention ; et
- la figure 14 est une vue en perspective de deux systèmes selon la figure 1 couplés au moyen de poignées de rotation.

La description est réalisée en relation avec des plans et photographies à une échelle réduite d'un système réel.

### DESCRIPTION DETAILLEE DE L'INVENTION

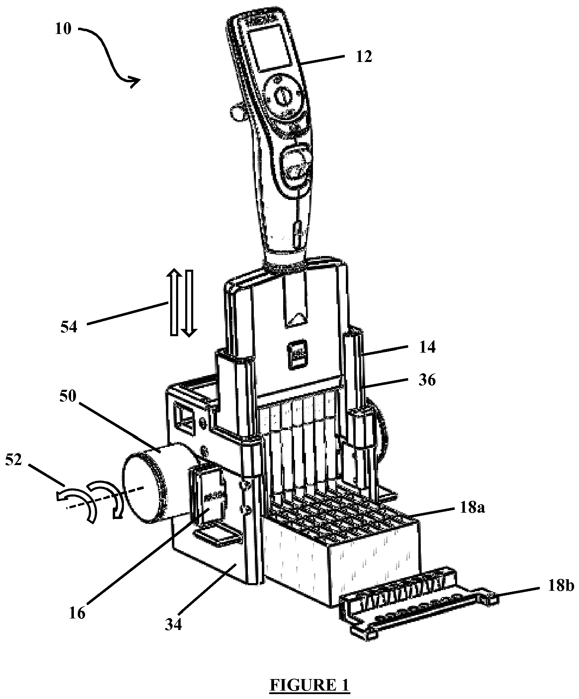

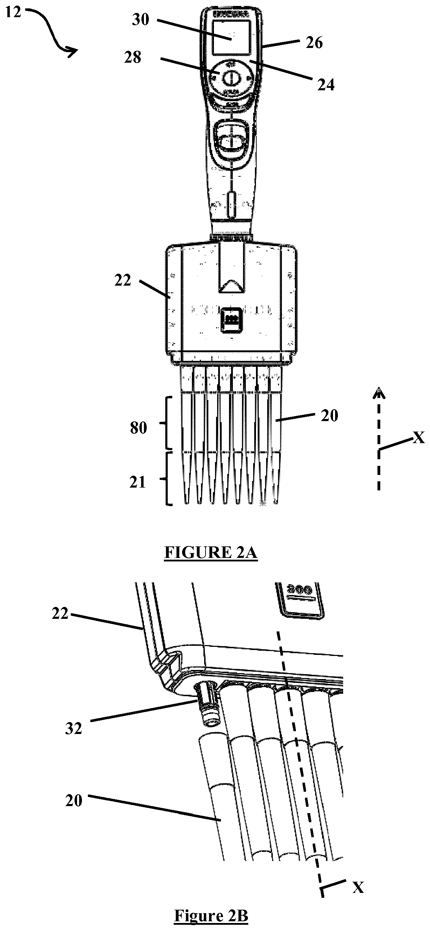

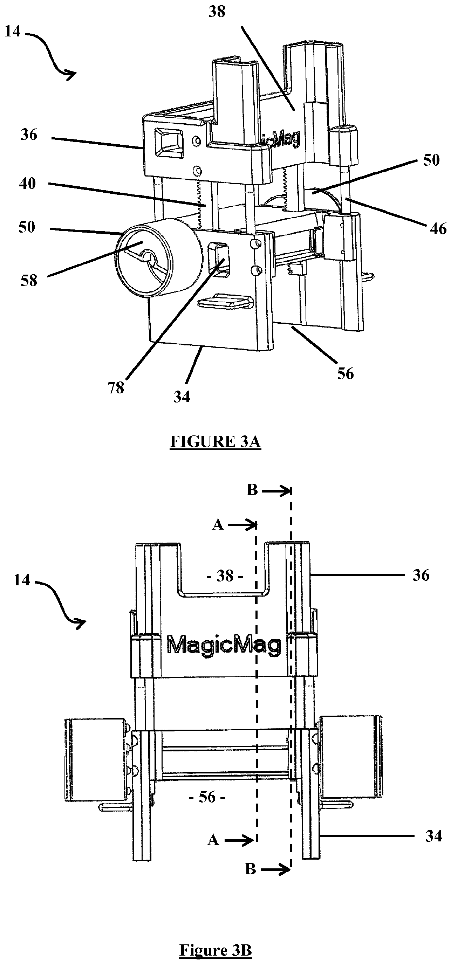

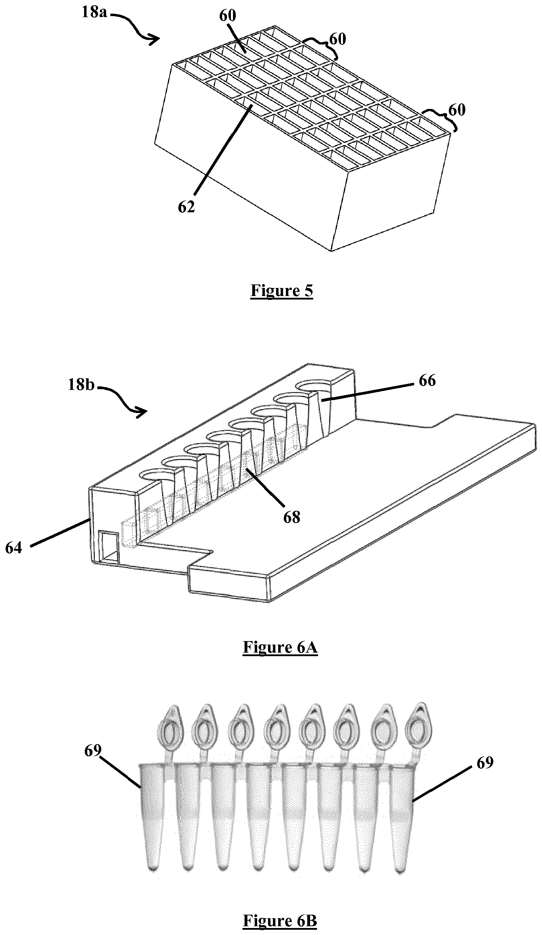

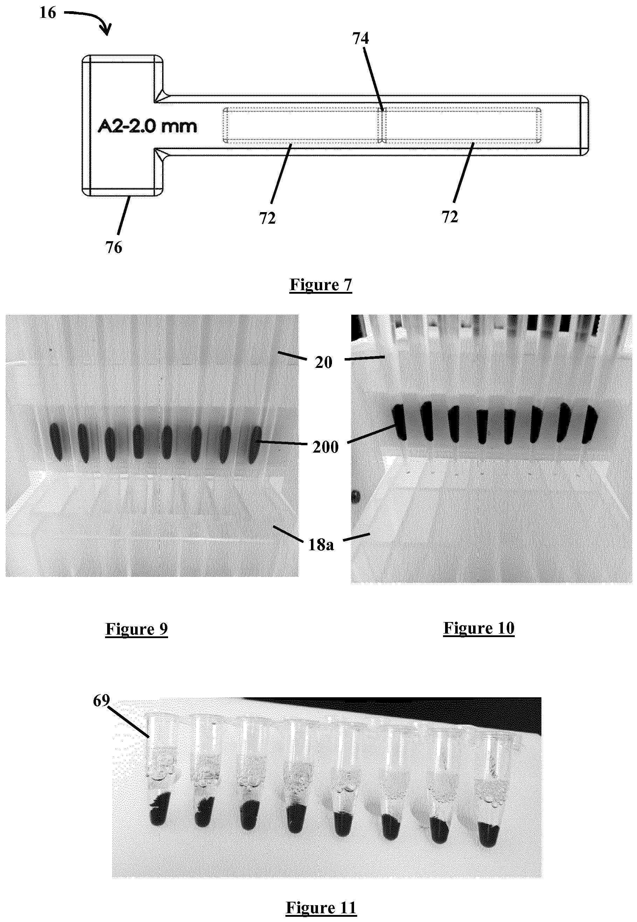

En se référant aux figures 1 à 7, un système **10** d'extraction (figure 1) de composants contenus dans un échantillon liquide comporte une pipette électronique **12** (figures 2), un porte-pipette **14** (figures 3) dans lequel la pipette **12** est logée, un ou plusieurs support de puits **18a, 18b** (figure 5 et 6) pouvant chacun être logé dans le porte-pipette **14,** et une première pièce aimantée **16** (figure 7).

La pipette **12,** qui est portable, comprend une rangée de cônes de pipettes **20,** et un corps **22** sur lequel sont montés les cônes **20** (figure 2A). Dans ce corps **20,** sont logés un circuit d'aspiration/refoulement de liquide dans les cônes **20** (par exemple un ensemble de pistons actionnés par un moteur électrique), et un circuit électronique de pilotage du circuit d'aspiration/refoulement. Le circuit électronique, qui comprend par exemple un microprocesseur et une ou plusieurs mémoires informatiques, est programmable, et embarque des instructions pour la mise en oeuvre d'un ou plusieurs protocoles de pipetage, chaque protocole comprenant une ou plusieurs étapes. Le circuit électronique comprend également une interface homme machine **24** logée dans une poignée **26** du corps **22,** l'interface comprenant un ensemble de boutons de sélection et de navigation **28** et un écran d'affichage **30** permettant la visualisation et la sélection de différents protocoles de pipetage enregistrés. L'utilisateur peut notamment programmer la pipette électronique **12,** e.g. en téléchargeant dans celle-ci des instructions depuis un ordinateur connecté à la pipette **12** au travers d'une liaison sans fil, par exemple Bluetooth. L'utilisateur peut également sélectionner, via l'interface **24,** un protocole préenregistré. La pipette **12** est par ailleurs apte à aspirer un volume prédéfini de liquide dans chacun des cônes **20,** refouler un volume prédéfini depuis chacun des cônes, mettre en oeuvre des cycles automatiques d'aspiration/refoulement de durée et de fréquence variable.

Comme illustré plus particulièrement à la figure 2B, les cônes **20,** de forme tubulaires et d'axe longitudinal X, sont amovibles en s'emboitant dans des lobes **32** faisant saillie du corps **22,** ce qui permet leur remplacement. Les cônes sont par ailleurs constitués d'un matériau plastique, par exemple en polypropylène, ce qui a pour effet de les rendre « transparent » à un champ magnétique et permet la capture de particules magnétiques, comme cela sera décrit plus en détails par la suite. Enfin, chaque cône **20** présente à son extrémité ouverte de pipetage un profil effilé **21,** ou « pointe ». Cette partie **21** a une section réduite dans un plan perpendiculaire à l'axe X, ce qui permet son introduction facilitée dans des récipients ou puits comme cela est connu en soi.

La pipette électronique **12** est par exemple le modèle « VIAFLO II 8 canaux » commercialisé par la société ©INTEGRA Biosciences AG, Suisse, modèle dont les éléments sont décrits dans les demandes de brevets US 2009/071266, US 2009/074622, US2011076205 et US 2008/095671.

Le porte-pipette **14** comporte quant à lui (figures 3A et 3B) un socle **34,** destiné à être posé sur un plan de travail (e.g. une table ou une paillasse de laboratoire), ainsi qu'une partie mobile **36** par rapport au socle **34.** La partie mobile **36,** également nommée « support de pipette », comporte un logement **38** dans lequel la pipette **12** peut être insérée de manière amovible et maintenue immobile comme illustré à la figure 1. Pour la translation du support de pipette **36** par rapport au socle **34,** le support **36** comporte une ou plusieurs crémaillères **40,** par exemple au nombre de deux, s'engrenant avec des roues dentées **42** montées sur un arbre **44** mobile en rotation et logé dans le socle **34.** Une ou plusieurs tiges **46** sont en outre fixées au support **36** (resp. dans le socle **34**) et coulissent dans des orifices du socle **34** (resp. dans le support **36**), afin de guider le support **36** dans son mouvement de translation. Une ou deux poignées de rotation **50** sont en outre fixées au bout de l'arbre **44** afin de permettre à l'utilisateur de facilement tourner ce dernier selon les flèches **52** (figure 1) et donc faire monter et descendre le support de pipette **36** tel qu'illustré par les flèches **54** (figure 1). Le mouvement de translation du support de pipette **36** par rapport au socle **34** est donc parallèle à l'axe X des cônes **20** lorsque la pipette **12** est placée dans le logement **38** du support **36,** et donc parallèle à la direction de la gravité lorsque le socle **34** est posé sur un plan de travail horizontal, de sorte que le support **36** « monte » ou « descend».

Le socle **34** est ouvert sur sa face avant **56** pour permettre l'introduction et le retrait des supports de puits **18a, 18b,** définissant ainsi un logement pour ces derniers. Ce logement est ouvert sur sa partie supérieure pour permettre aux cônes **20** de la pipette **12** d'atteindre lesdits supports de puits lorsque la pipette descend. Ainsi, comme décrit plus en détail ci-après, la pipette **12** peut prendre plusieurs positions par rapport au socle **34,** et donc par rapport à un support de puits **18a, 18b** logé dans ce dernier. En particulier, la pipette **12** peut prendre une position dans laquelle les pointes **21** des cônes **20** trempent dans des puits du support **18a, 18b,** et au moins une position dans laquelle les pointes **21** ne trempent pas dans les puits, et sont à distance de ces derniers de manière à permettre la manipulation des supports de puits par l'utilisateur et la capture de particules magnétiques dans une position centrale des cônes **20.**

En se référant aux figures 5 et 6, les supports de puits peuvent prendre plusieurs formes en fonction de l'extraction souhaitée. Notamment, un support de puits est une plaque **18a** compartimentée (figure 5), usuellement nommée « microplaque » de type « DeepWell ». Ce type de plaque comporte des rangées **60** de puits **52** dans lesquelles peut plonger la rangée de cônes de pipette **20.** Chaque rangée **60** peut ainsi recevoir un liquide particulier utilisé lors de l'étape d'extraction mise en oeuvre par le système **10.** Le passage d'une rangée **60** à l'autre est alors réalisé simplement par l'utilisateur qui met à l'aplomb des cônes de pipettes **20** la rangée **60** particulière contenant le liquide nécessaire à l'étape devant être mise en oeuvre.

Un autre support de puits, décrit à la figure 6A, est aimanté et spécifiquement conçu pour la migration de particules magnétiques depuis les pointes **21** des cônes de pipettes **20** dans des puits. Le support **18b** comprend à cet effet un corps **64** dans lequel est formée une rangée de de logement **66** pouvant recevoir la rangée de cône de pipette **20,** et dans lequel est logée une seconde pièce aimantée **68** comprenant un ou plusieurs aimants permanents, par exemple un aimant permanent à proximité de chacun des puits **66.** La pièce aimantée **68** est placée sous les puits **66** ou en face de leur portions inférieures comme illustré. Ainsi, les pointes **21** des cônes de pipettes **20** sont placées au-dessus de la pièce aimantée **68** lorsque lesdites pointes sont plongées dans les puits **66.** Enfin, le support **18b** sert de portoir magnétique revevant de manière amovible des tubes **69** dans les logements **66,** par exemple des tubes d'élution PCR tels qu'illustrés à la figure 6B, à des fins par exemple de transfert ultérieur du produit de l'extraction récupéré dans ceux-ci.

La première pièce aimantée **16,** dont la fonction est de capturer des particules aimantées dans les cônes **20** d'une manière décrite plus en détail par la suite, comprend quant à elle un ou plusieurs aimants permanents **72,** avantageusement une rangée d'aimants permanents séparés les uns des autres par des espaces **74,** et encore plus avantageusement un aimant permanent en face de chaque cône de pipette **20** lorsque la pièce **16** est entièrement logée dans le socle **34.** La pièce **16** comprend en outre une poignée **76** pour une meilleure préhension par l'utilisateur.

Un logement **78** pour la réception de la pièce aimantée **16** est prévu dans le socle **34,** le logement **78** étant placé de manière à ce que la pièce **16** soit en face des cônes de pipette **20** au-dessus de leur pointe **21,** et de préférence face à une zone centrale **80** à une hauteur supérieure au puits , lorsque les pointes **21** plongent dans les puits maintenu dans un support de puits. De cette manière, les particules sont capturées dans un volume du cône suffisamment grand pour ne pas former de bouchons dans les cônes.

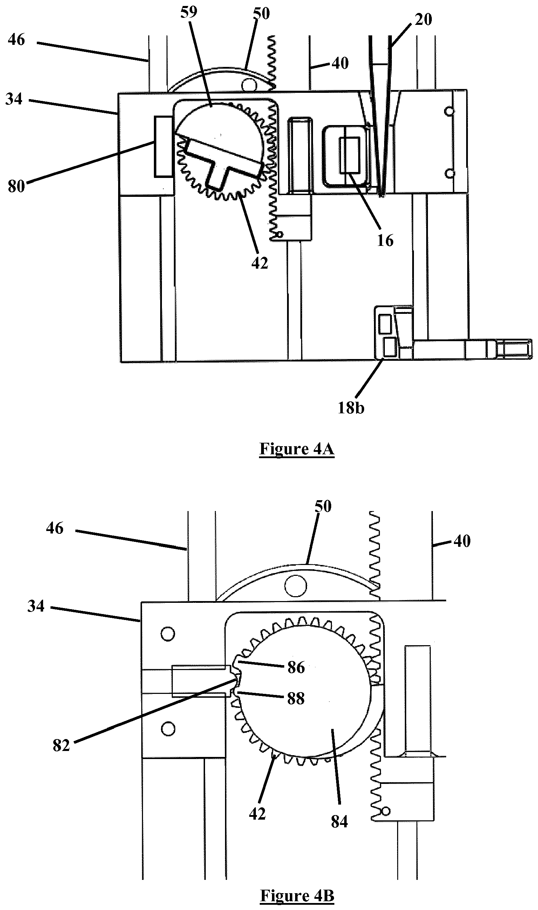

Le porte-pipette **14** comporte également des moyens permettant de maîtriser la vitesse de remontée du support **36.** En particulier, la crémaillère et la roue dentée sont conçus pour qu'un demi-tour (180°) de la roue **50** permette de parcourir l'ensemble de la crémaillère, et une masselotte **58** intégrée dans chacune des poignées **50** de manière désaxée par rapport à l'arbre **44.** Ces masselottes, sous leur poids et l'effet de levier associé, génèrent un couple de rotation mettant en rotation l'arbre **44** tout en limitant le couple transmis à la main par l'utilisateur. De manière avantageuse, comme illustré à la figure 4A, une partie substantielle de l'arbre **44** est formé également d'une masselotte demi-cylindrique dans le même but. Cette assistance mécanique aide à soulever le support-de pipette, et limite donc les troubles musculo-squelettiques, et met en oeuvre un frein qui permet à l'utilisateur de contrôler avec plus de précision la vitesse de montée et de descente du support **36.**

D'autres mécanismes de contrôle de la vitesse du support **36** peuvent être prévus, notamment un freinage magnétique. Par exemple, en se référant à la figure 4A, la masselotte **59** comprend un matériau aimantable (e.g. acier ou équivalent) et une troisième pièce aimantée **80** (parallélépipédique ou en forme d'arc de cercle concentrique à l'arbre **44**) est logée dans le socle **34,** de préférence à l'opposé de la pièce aimantée **16** vis-à-vis de l'arbre **44** pour ne pas perturber l'extraction. Lorsque la masselotte 59 de l'arbre passe devant la pièce aimantée **80,** le mouvement de rotation de l'arbre **44** est ralenti en raison du couple de freinage engendré. Ceci permet d'une part de compenser l'effort pour soulever la pipette lors de la montée (en jouant le rôle d'assistance pour l'utilisateur), et d'autre part de maitriser la vitesse de remontée de la pipette lorsque l'on veut faire descendre les particules magnétiques en bas des cônes de pipette, comme cela sera décrit ci-dessous.

Un mécanisme de butée est également avantageusement prévu comme illustré à la figure 4B. Dans cette variante, une roue **84** en matériau déformable (e.g. en élastomère) est montée sur l'arbre **44** et comporte deux dents **86, 88.** Une protubérance **82,** par exemple hémisphérique, fait par ailleurs saillie du socle **84** en face de la roue **84.** Lorsque l'utilisateur remonte la pipette **12** en actionnant la roue **50,** la première dent **86** rencontre la protubérance **82.** En augmentant le coupe appliqué à la roue **50,** la dent **86** se plie, passe la protubérance **82,** et reprend sa forme. Dans cette position, la dent **86** peut alors reposer sur la protubérance **82,** la dureté de cette dent étant choisi pour qu'elle ne se plie pas sous l'action du poids de la pipette **12** et du porte-pipette **36.** La pipette est ainsi bloquée en position haute, l'utilisateur pouvant donc relâcher la roue **50.** La deuxième dent **88,** de plus grande dimension (e.g. longueur et/ou largeur) nécessite pour son passage un couple beaucoup important, et définit ainsi une butée pour éviter que le porte-pipette **36** ne se déboite du socle **34,** à moins que l'utilisateur déploie une force apte à casser cette dent. En variante, la protubérance **82** est remplacée par une butée comportant une bille montée sur ressort dans un logement du socle. La roue **84** peut ainsi être réalisée un matériau dur. L'action de la première dent **86** a alors pour effet de pousser la bille dans son logement, permettant ainsi le passage de la dent **86..**

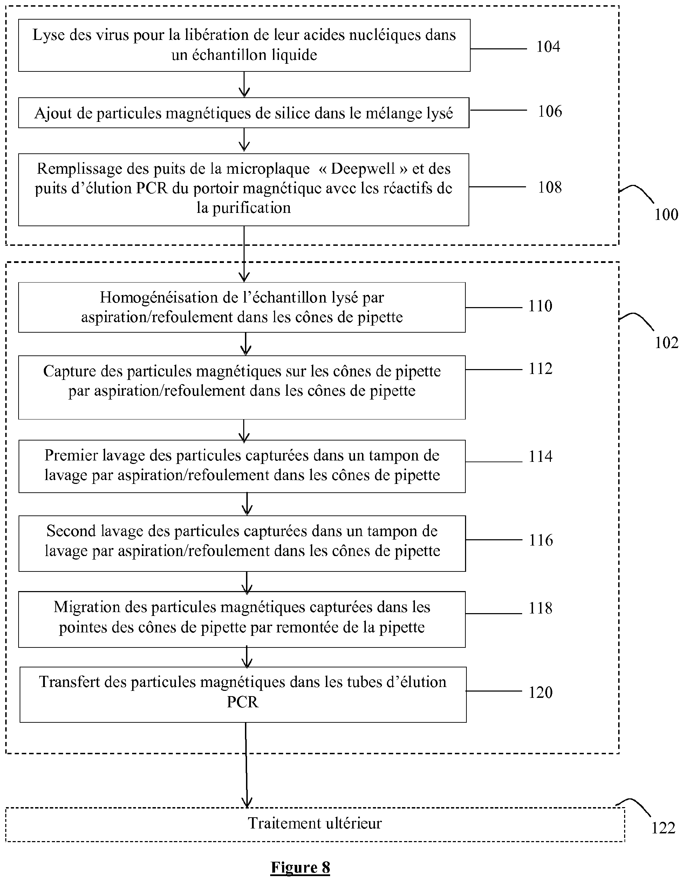

Il est à présent décrit un procédé d'extraction de composants contenus dans un échantillon liquide à l'aide de particules magnétiques, ce procédé étant mis en oeuvre à l'aide du système venant d'être décrit. Le procédé se fonde sur l'association du porte-pipette, de la pipette électronique programmable et de cônes de pipette (e.g. d'un volume de 1250 µL) afin de réaliser les différentes étapes de capture, de lavage et d'élution de particules magnétiques pour traiter un volume d'échantillon par cône de pipette compris entre 1 mL et 5 mL. La capture des particules magnétiques s'effectue séquentiellement dans les cônes de pipette au cours de cycles d'aspiration/refoulement sur l'ensemble du volume d'échantillon à traiter. A titre d'exemple, il est décrit en relation avec l'organigramme de la figure 8 un procédé de purification d'acides nucléiques viraux utilisant la chimie NucliSENS©, à savoir une extraction des acides nucléiques à l'aide de particules de silice magnétiques.

Le procédé débute par une étape **100** de préparation des différents échantillons et réactifs nécessaires à la purification, suivie de ladite purification en **102.**

Notamment, la préparation **100** consiste, en **104,** à mélanger l'échantillon biologique comprenant des virus dont on souhaite extraire les acides nucléiques, avec un réactif de lyse chimique des virus (e.g. le réactif de lyse « Nuclisens miniMAG » de bioMérieux, de référence 200292, ou le réactif de lyse « Nuclisens easyMAG » de bioMérieux, de référence 280130), à raison de deux volumes de réactif de lyse pour un volume d'échantillon. Le mélange est ensuite chauffé pendant 30 minutes à 56°C, libérant ainsi les acides nucléiques des virus d'une manière connue en soi. Des particules de silice magnétiques, ayant pour propriété de se lier avec des acides nucléiques, sont alors introduites, en **106,** dans l'échantillon lysé.

La préparation **100** se poursuit, en **108,** par le remplissage de la microplaque **18a,** ayant des puits **62** de 5mL, et des tubes d'élution PCR **69** de 0,2 mL du portoir magnétique **18b** de sorte que :
- chaque puits de la première rangée de la microplaque **18a** est rempli de l'échantillon lysé comprenant les particules de silice, ci-après « échantillon lysé ». Le volume total dans chaque puits de la première rangée est de préférence supérieur à 1,5 mL en raison de l'utilisation de la microplaque Deepwell 5 mL et des volumes manipulés par la pipette électronique ;
- chaque puits **66** de la deuxième rangée de la microplaque **18a** est rempli de 1250 µL de tampon de lavage (e.g. le « NucliSENS easyMAG Extraction Buffer n°2 » de bioMérieux de référence bMx 280131) ;
- chaque puits **66** de la troisième rangée de la microplaque **18a** est rempli de 1250 µL de tampon de lavage (e.g. le « NucliSENS easyMAG Extraction Buffer n°2 » de bioMérieux de référence bMx 280131) ;
- chaque tube d'élution PCR **69** logé dans le portoir magnétique **18b** est rempli d'un volume de 100 µL de tampon d'élution (e.g. le « NucliSENS easyMAG Extraction Buffer n°3 » de bioMérieux, de référence 280132).

L'utilisateur place alors :
- la pipette électronique **12,** avec sa rangée de cônes **20,** dans le logement **38** du porte-pipette **14,** en position relevée pour permettre l'introduction de la plaque **18a** ; et
- la plaque **18a** dans le logement **56** du socle **34** avec la première rangée de puits comprenant l'échantillon lysé à l'aplomb des cônes **20.**

L'extraction **102** débute par l'homogénéisation de l'échantillon lysé. Pour se faire, la pièce aimantée **16** n'est pas placée dans le socle **34** et n'interfère donc pas avec les cônes **20.** L'utilisateur tourne une des roues **50** de manière à plonger les pointes **21** des cônes **20** dans la rangée de puits de la plaque **18a** comprenant l'échantillon lysé. Puis, il sélectionne à l'aide de l'interface **24** de la pipette **12** un premier protocole de pipetage comprenant au moins une phase d'aspiration/refoulement de l'échantillon lysé dans les cônes **20,** et lance le protocole sélectionné. Ces phases (e.g. au nombre de deux) comprennent chacune au moins un cycle d'aspiration/refoulement (e.g. cinq cycles) suivi d'une durée d'attente de plusieurs minutes, par exemple 5 minutes. Au sens de l'invention, un cycle d'aspiration et de refoulement consiste à remplir au moins au trois quart, par exemple complètement, les cônes puis à les vider complètement, à moins qu'il ne soit spécifié autrement par le programme.

Une fois l'homogénéisation terminée, les cônes **20** sont vides et leurs pointes **21** plongent dans les puits contenant l'échantillon lysé. La purification **102** se poursuit par la capture, en **112,** des particules de silice de l'échantillon lysé sur la paroi interne des cônes **20.** A cet effet, l'utilisateur place la pièce aimantée **16** dans le logement **78** du socle **34,** sélectionne à l'aide de l'interface **24** de la pipette **12** un second protocole de pipetage puis lance le protocole sélectionné. Le second protocole comprend une pluralité de cycles d'aspiration/attente/refoulement, e.g. une dizaine de cycles, une aspiration étant séparée d'un refoulement de quelques secondes, e.g. une dizaine de secondes. A chaque aspiration et chaque refoulement, une partie des particules contenues dans l'échantillon lysé est capturée sur la paroi des cônes de pipette grâce au champ magnétique produit par la pièce aimantée **16.** Les particules magnétiques, et donc également leurs acides nucléiques liés, sont ainsi capturées sous le forme de culots de particules **100** en face de la pièce aimantée **16,** et de préférence sur une zone centrale à mi-hauteur des cônes **20,** comme illustré à la figure 9.

Une fois la capture terminée, l'échantillon lysé ayant été refoulé complètement des cônes **20** et la pièce aimantée **16** étant toujours en place, la purification **102** se poursuit par une première étape de lavage **114.** A cette fin, l'utilisateur soulève le porte-pipette **14** (resp. remonte la pipette **12**) de manière à libérer la plaque **18a** des cônes **20,** aligne la deuxième rangée de la plaque **18a** avec la rangée de cône **20** puis replace le porte-pipette (resp. fait descendre la pipette) de manière à faire tremper les pointes **21** des cônes dans les puits de la plaque **18a.** L'utilisateur sélectionne ensuite, à l'aide de l'interface **24,** un troisième protocole de pipetage comprenant au moins une phase d'aspiration/refoulement de l'échantillon lysé dans les cônes **20,** puis lance le protocole sélectionné. Le troisième protocole est par exemple identique au premier protocole. Le passage répété du tampon de lavage sur les culots de particules permet ainsi de laver ces derniers. Cette étape de lavage est avantageusement complétée, ou mise en oeuvre de manière conjointe, à une modulation du champ magnétique capturant les particules sur les cônes. Par exemple, l'utilisateur fait remonter et descendre la pipette **12,** ce qui a pour effet de déplacer les culots de particules sur les cônes, ou bien la pièce aimantée **16** comprend un train d'aimants permanents et l'utilisateur fait coulisser dans un mouvement de va-et-vient la pièce aimantée **16** de son logement **78,** de sorte que l'intensité et les lignes de champs magnétiques capturant les culots varient, tout en conservant les particules capturées sur les cônes. La modulation du champ magnétique a ainsi pour effet de réorganiser les culots lors du lavage, et augmenter l'efficacité de celui-ci.

Un second lavage est ensuite mis en oeuvre en **116** à l'aide du tampon de lavage de la troisième rangée de la plaque **18a.** Par exemple, les cônes sont complètement vidés du premier tampon de lavage, puis une second lavage identique au premier lavage est réalisé.

A la suite de ce second lavage, une étape de migration **118** des culots de particules **200** dans les pointes **21** des cônes **20** est mise en oeuvre. Pour ce faire, les cônes **20** restent préférentiellement remplis du second tampon de lavage pour faciliter la glisse des culots **200** et restent alignés avec la seconde rangée de la plaque **18a.** L'utilisateur tourne alors une des roues **50** pour faire remonter la pipette **12.** La pièce aimantée **16** étant solidaire du socle **34,** les culots restent donc immobiles par rapport à cette dernière et migrent vers les pointes **21** en glissant sur les parois des cônes **20** à mesure de la remontée de la pipette. L'utilisateur stoppe la remontée de la pipette **12** une fois les culots **200** dans les pointes **21,** comme illustré à la figure 10, à une distance moyenne de quelques millimètres, e.g. 8 mm, des extrémités ouvertes des cônes. Dans cette position, l'utilisateur sélectionne et lance ensuite à l'aide de l'interface **24** le refoulement du tampon de lavage contenu dans les cônes **20** dans les puits de la plaque **18a.** Optionnellement, une des phases de lavage, ou une phase de lavage supplémentaire consiste à retirer la pièce aimantée de manière à libérer les particules magnétiques et réaliser un lavage tout en brassant les particules dans la solution de lavage par des cycles d'aspiration et de refoulement. Les particules sont ensuite capturées une nouvelle fois en replaçant la pièce aimantée et en procédant à des cycles d'aspiration et de refoulement tel que décrit précédemment.

La purification **102** se termine par une étape **120** de transfert dans les tubes d'élution PCR **69** des particules magnétiques présentes dans les pointes **21** des cônes **20.** A cet effet, l'utilisateur soulève le porte-pipette **14,** ôte la plaques **18a,** place le portoir magnétique **18b** dans le logement **56** de manière à aligner les tubes PCR **69** avec la rangée de cônes **20,** repose le porte-pipette **14** et retire la pièce aimantée **16** du socle **14** afin de libérer les particules magnétiques capturées des cônes. Une fois les pointes **21** plongées dans les tubes **69,** l'utilisateur sélectionne, à l'aide de l'interface **24,** un quatrième protocole de pipetage, puis lance le protocole sélectionné. Une première variante de ce protocole consiste en des cycles d'aspiration et de refoulement du tampon d'élution dans les pointes **21** des cônes **20,** ce qui permet la remise en suspension des particules magnétiques en cassant les culots de particules. Par ailleurs, la fréquence choisie pour les cycles permet, à chaque refoulement dans les tubes **69,** à une parties des particules magnétiques d'être capturées dans les tubes **69** grâce au champ magnétique de la pièce aimantée **68** logée dans le portoir **64.** De plus ces cycles permettent de « rincer » les pointes **21** pour récupérer des particules adhérant aux parois des cônes. Dans une deuxième variante du protocole, des cycles d'aspiration et de refoulement sont tout d'abord mis en oeuvre à une fréquence plus élevée de façon à brasser plus vigoureusement le tampon et les particules, et donc d'obtenir une homogénéisation accélérée facilitant le transfert dans les tubes d'élution **69.** L'étape de transfert se termine par le refoulement complet du tampon d'élution dans les tubes **69.** Sous l'effet du champ magnétique du portoir **64,** les particules magnétiques sont alors définitivement séparées du tampon d'élution, comme illustré à la figure 11. L'utilisateur peut ainsi récupérer les tubes **69** pour un traitement ultérieur, en particulier l'élution des acides nucléiques par chauffage, d'une manière connue en soi.

Dans le mode de réalisation du porte-pipette décrit précédemment, la pièce aimantée **16** est logée dans le socle **34.** Aussi, lorsque l'utilisateur souhaite avancer la plaque **18a,** il peut remonter la pipette suffisamment haut pour procéder à cette opération. Ceci provoque, comme pour la migration des particules vers les pointes, le déplacement des culots **200** sur les parois des cônes **20,** ce qui présente l'avantage de « réorganiser » les culots qui peuvent rouler sur eux-mêmes. L'efficacité du lavage s'en trouve ainsi renforcée. Par contre, cela implique que l'utilisateur prend garde à ne jamais trop remonter la pipette afin d'éviter que les culots sortent des cônes. Pour ce faire, l'utilisateur peut par exemple soulever ou basculer le porte pipette pour conserver les culots à distance des ouvertures des cônes. Cette option, qui nécessite le soulèvement répété d'un dispositif dont le poids peut être important, peut cependant entraîner à long terme des troubles musculo-squelettiques. En outre, l'utilisateur doit encore prendre garde de ne pas trop soulever le porte-pipette afin d'éviter que les culots sorte des cônes.

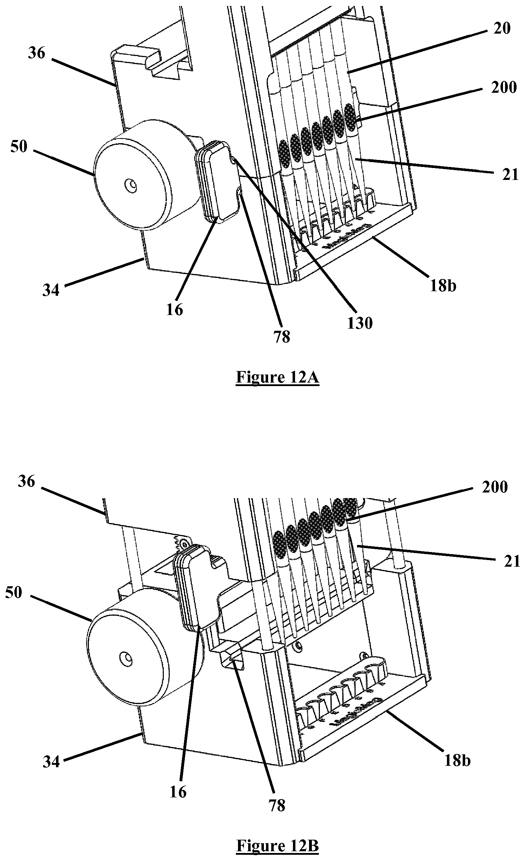

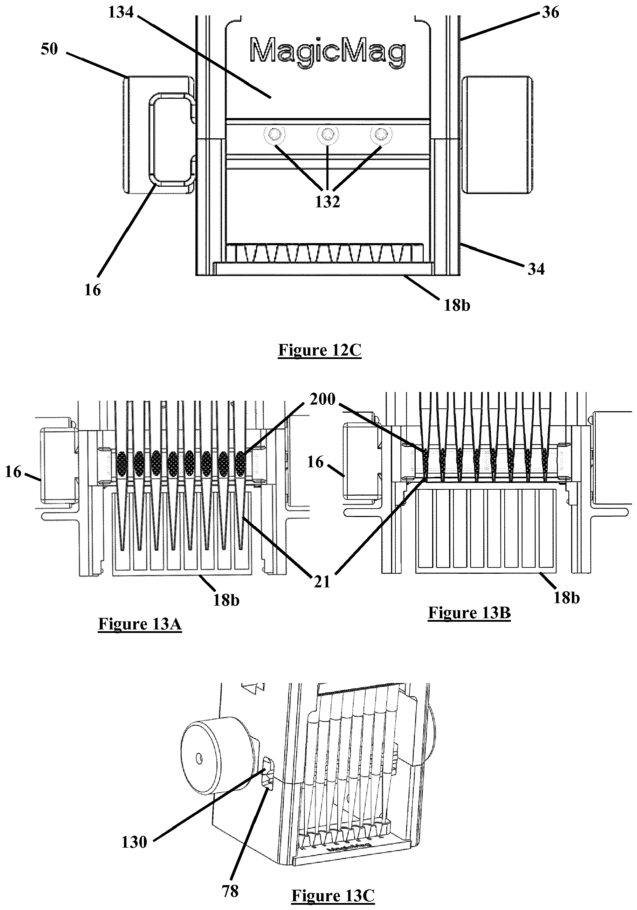

Une second mode de réalisation du porte-pipette selon l'invention permet la manipulation des plaques **18a, 18b** en remontant uniquement la pipette, et donc en évitant de soulever le porte-pipette **14,** tout en garantissant que les culots de particules restent à distance des pointes **21** des cônes. Ce second mode de réalisation, ainsi que les variations induites sur le procédé venant d'être décrit, sont illustrés aux figures 12 et 13.

Plus particulièrement, le second mode de réalisation diffère du premier mode de réalisation par les moyens de réception de la pièce aimantée **16** dans le porte-pipette **14.** Notamment, le socle **34** comprend le logement **78** pour l'insertion et le retrait de la pièce aimantée **16** comme décrit précédemment et le logement **78** est ouvert dans sa partie supérieure **130** pour permettre également l'insertion et le retrait de la pièce **16** verticalement dans le logement **78.** Le support de pipette **36** comprend en outre des moyens pour fixer la pièce aimantée **16** à l'aplomb du logement ouvert **78,** en particulier un ou plusieurs plots **132** en matériau aimantable (e.g. en acier) fixés sur une paroi arrière **134** du support de pipette mobile **36** (figure 12C). De cette manière, la pièce aimantée **16** est solidaire du support mobile **36,** et reste en face des cônes **20** lorsque l'utilisateur remonte et descend la pipette (en particulier lors des phases de lavage), comme illustrée aux figures 12B à 13A.

Pour réaliser la migration des culots **200** dans les pointes des cônes **20,** l'utilisateur désolidarise la pièce aimantée **16** du support de pipette **36,** en appliquant en simple pression vers le bas sur la poignée **78** de la pièce **16** et remonte la pipette **12.** La pièce aimantée **16** se détache des plots **134,** reste donc dans le logement **78** du socle et est donc solidaire du socle **34,** induisant la migration des culots **200** dans les pointes **21** des cônes comme décrit précédemment (figures 13A et 13B). Une fois la pipette remontée, l'utilisateur remplace la plaque **18a** par le portoir **18b** muni des tubes d'élution PCR, retire la pièce aimantée **16** et redescente la pipette (figure 13C, tubes **69** non représentés). En variante, le support de pipette **36** peut comporter un logement analogue au logement **78** du socle dans lequel l'utilisateur glisse la pièce aimantée notamment pour les phases de lavage.

Il a été décrit un procédé d'extraction particulier. La présente invention s'applique cependant à tout type de capture de particules magnétiques et à tout type de séquence de pipetage. De même, il a été décrit une pipette ayant 8 canaux d'un volume particulier. La pipette peut comprend un nombre quelconques de canaux de volume quelconque en fonction de l'application visée.

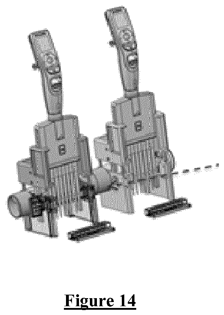

Afin d'augmenter le nombre d'échantillons traités, deux systèmes d'extraction selon l'invention peuvent être couplés, comme illustré à la figure 14. Par exemple, les poignées de rotation **50** peuvent s'emboiter de sorte que deux extractions peuvent être menées simultanément, l'utilisateur remontant et descendant les pipettes **12** en même temps. A cette fin, les pipettes peuvent également être synchronisées, une pipette commandant par exemple l'autre pipette.

De même, il a été décrit un système d'extraction portable et semi-automatisé, particulièrement adapté aux laboratoires d'analyse ayant un nombre limité d'extractions à réaliser au quotidien. L'invention peut cependant être automatisée. Par exemple, la pipette est intégré à un automate qui comprend des mécanismes programmables de montée et de descente de la pipette et de mouvement d'aimant (ou d'activation/désactivation d'électroaimants).

La présente invention répond à une problématique de polyvalence pour l'utilisation de différentes techniques de capture magnétique (purification des acides nucléiques, Immuno-concentration magnétiques,...). Le système selon l'invention, évolutif et modulable, permet :
- la réalisation d'étapes de capture/lavage/élution de particules magnétiques en utilisant un système autonome constitué par l'association d'une pipette électronique programmable et d'un support permettant la réalisation des différentes étapes précitées ;
- le traitement d'un nombre d'échantillon de 1 à 8 en fonction de la configuration de la pipette électronique utilisée ;
- la parallélisation du traitement des échantillons dans le cadre défini ci-dessus avec un système semi-automatique ;
- l'association de 2 systèmes si besoin d'augmenter le nombre d'échantillon à traiter ;1
- les étapes d'élution peuvent être réalisées dans différents types de tubes : tubes PCR 0.2 mL pour récupération des particules de silice magnétiques lorsqu'il s'agit de capture des acides nucléiques (e.g. chimie NucliSENS©) ou bien tubes de bead-beating dans le cas de la récupération de particules magnétiques ayant servi à la récupération de pathogènes (Immuno-Concentration Magnétique). A cette fin, le système permet la réalisation d'étapes de capture/concentration des pathogènes sur les particules magnétiques et leur lyse in-situ à l'aide de billes de céramique / verre (e.g. procédé de type CapLyse©).

## Revendications

1. Procédé d'extraction de composants contenus dans un échantillon biologique sous forme liquide, lesdits composants étant aptes à se fixer sur des particules magnétiques, le procédé comprenant :
- une phase (106) de mélange de l'échantillon avec les particules magnétiques;
- une phase (110) d'aspiration du mélange depuis un puits dans un cône de pipette (20) tubulaire comprenant une pointe (21) destinée au pipetage de liquide;
- une phase de capture (112) des particules magnétiques sur une paroi interne du cône de pipette (20) :
∘ en appliquant un premier champ magnétique (16) au cône de pipette (20), ledit champ étant apte à attirer et maintenir les particules magnétiques dans une zone prédéterminée du cône de pipette (20), dite de « capture », au-dessus de la pointe de celui-ci ;
∘ et en appliquant au moins un cycle d'aspiration et de refoulement du mélange contenu dans le cône de pipette (20) dans un puits (62);
- au moins une phase (114, 116) de lavage des particules capturées sur la paroi interne du cône de pipette (20) en :
∘ refoulant le mélange contenu dans le cône de pipette (20) ; et
∘ en appliquant depuis un puits (62) contenant une solution de lavage au moins un cycle d'aspiration et de refoulement de la solution de lavage dans le cône de pipette (20) ;
- une phase (118) de migration des particules magnétiques sur la paroi interne du cône de pipette (20), depuis la zone de capture jusqu'à la pointe (21) du cône de pipette (20), en réalisant un déplacement relatif du cône de pipette (20) par rapport au premier champ magnétique (16) ;
- et une phase (120) de transfert desdites particules magnétiques ayant migré dans la pointe (21) du cône de pipette (20) dans un puits de récupération (69) contenant une solution.

2. Procédé selon la revendication 1, dans lequel le déplacement du premier champ magnétique (16) consiste à déplacer le cône de pipette (20) parallèlement à un axe longitudinal (X) dudit cône (20), et à conserver constant le premier champ magnétique (16), l'axe longitudinal (X) du cône de pipette restant à égale distance du premier champ magnétique (16) lors du déplacement du cône de pipette (20).

3. Procédé selon la revendication 1 ou 2, dans lequel la phase (120) de transfert comprend :
- le placement de la pointe (21) du cône de pipette (20) dans le puits de récupération (69);
- et l'application d'un second champ magnétique (68) depuis le fond du puits de récupération (69) de manière à faire migrer dans le puits de récupération (69) les particules magnétiques contenues dans la pointe (21) du cône de pipette (20).

4. Procédé selon la revendication 3, dans lequel le second champ magnétique (68) est produit par un aimant positionné partiellement ou entièrement sous la pointe du cône de pipette.

5. Procédé selon la revendication 3 ou 4, dans lequel le premier champ magnétique appliqué au cône de pipette est désactivé lors de l'application du second champ magnétique.

6. Procédé selon l'une quelconque des revendications 3 à 5, dans lequel la phase (120) de transfert comporte la désactivation du premier champ magnétique (16) suivie de l'application de cycles d'aspiration et de refoulement de la solution du puits de récupération (69) dans la pointe (21) du cône de pipette, ladite application comprenant :
- une première phase d'application des cycles à une première fréquence ;
- suivie d'une deuxième phase d'application des cycles à une deuxième fréquence, inférieure à la première fréquence.

7. Procédé selon l'une quelconque des revendications précédentes, comprenant, préalablement à la phase (112) de capture, une phase de brassage du mélange contenu dans le cône de pipette par l'application d'au moins un cycle d'aspiration et de refoulement dudit mélange dans le cône de pipette.

8. Procédé selon quelconque des revendications précédentes, comprenant, préalablement à la phase (120) de transfert, au moins une phase de lavage des particules capturées sur la paroi interne du cône de pipette en :
- en désactivant le champ magnétique ;
- en appliquant depuis un puits contenant une solution de lavage au moins un cycle d'aspiration et de refoulement de la solution de lavage dans le cône de pipette ;
- en appliquant une deuxième phase de capture sur une paroi interne du cône de pipette :
∘ en appliquant le premier champ magnétique au cône de pipette
∘ et en appliquant au moins un cycle d'aspiration et de refoulement du mélange contenu dans le cône de pipette dans le puits contenant la solution de lavage.

9. Procédé selon quelconque des revendications précédentes, comprenant, préalablement à la phase (120) de transfert, au moins une phase de lavage des particules capturées sur la paroi interne du cône de pipette en :
- en aspirant de la solution de lavage dans ledit cône de pipette ;
- puis en modulant le premier champ magnétique appliqué aux particules magnétiques pour capturer celles-ci sur la paroi interne du cône de pipette ;
- puis en refoulant le liquide de lavage du cône de pipette.

10. Procédé selon la revendication 9, dans lequel la modulation du premier champ magnétique est réalisé :
- en déplaçant le cône de pipette parallèlement à un axe longitudinal dudit cône, et en conservant constant le premier champ magnétique ;
- et/ou en faisant défiler des aimants espacés les uns des autres devant les particules capturées.

11. Procédé selon l'une quelconque des revendications précédentes, dans lequel le volume du cône de pipette est au moins dix fois supérieur au volume du puits de récupération.

12. Procédé selon l'une quelconque des revendications précédentes, dans lequel le volume du mélange est au moins trois fois supérieur au volume du cône de pipette.

13. Procédé selon l'une quelconque des revendications précédentes, dans lequel les composants appartiennent au groupe formé des acides nucléiques, des microorganismes, des protéines, et des peptides.

14. Porte-pipette (14) comprenant :
- un socle (34) ;
- un évidement (56) formé dans le socle (34) et apte à loger de manière amovible un support de puits (18a, 18b) ;
- un support de pipette (36) comprenant un premier logement (38) dans lequel est apte à être insérée une pipette (12) équipée d'au moins un cône de pipette tubulaire (20) comprenant une pointe (21) destinée au pipetage de liquide, le premier logement (38) étant ouvert sur l'évidement (56) du socle, le support de pipette (36) étant mobile en translation par rapport au socle (34) selon une direction parallèle à un axe (X) des cônes de pipette (20) et mobile entre une première position dans laquelle la pointe (21) de chaque cône de pipette est logée dans un puits (62, 69) du support de puits (18a, 18b) et au moins une deuxième position dans laquelle ladite pointe (21) est en dehors dudit puits (62, 69) ;
- un second logement (78) apte à loger de manière amovible une pièce aimantée (16), le second logement (78) faisant face à chacun des cônes de pipette (20) dans une position au-dessus de la pointe de celui-ci lorsque le support de pipette (36) est dans la première position, et le second logement (78) faisant face à la pointe du cône de pipette (20) lorsque le support de pipette (36) est dans la deuxième position.

15. Porte-pipette selon la revendication 14, dans lequel le premier logement (38) comprend une ouverture pour l'insertion et le retrait frontaux de la pipette (12) dans le premier logement (38).

16. Porte-pipette selon la revendication 14 ou 15, dans lequel le second logement (78) est réalisé dans le socle (36).

17. Porte-pipette selon l'une quelconque des revendications 14 à 15, comprenant un troisième logement dans le support de pipette (36) dans lequel la pièce aimantée (16) est apte à être logé de manière amovible pour faire face à chaque cône de pipette (20) à une position au-dessus de la pointe (21) dudit cône lorsque le support de pipette (36) est dans la deuxième position.

18. Porte-pipette selon la revendication 17, dans lequel le second et troisième logements communiquent, et dans lequel le support de pipette comprend des moyens aptes à maintenir de manière amovible la pièce aimantée dans le troisième logement.

19. Porte-pipette selon l'une quelconque des revendications 14 à 18, dans lequel :
- le socle (34) comprend au moins une roue dentée (42) mobile en rotation ;
- et le support de pipette comprend une crémaillère (40) coopérant avec la roue dentée (42) pour translater le support de pipette (36) par rapport au socle (34) lors de la rotation de la roue dentée.

20. Porte-pipette selon la revendication 19, comprenant un dispositif de verrouillage (et de déverrouillage du support de pipette (82) au moins dans la première position.

21. Porte-pipette selon l'une des revendication 19 ou 20, comprenant au moins une poignée solidaire (50) de la roue dentée pour faire tourner celle-ci et apte à se fixer de manière amovible à une poignée solidaire (50) de la roue dentée d'un autre porte-pipette.

22. Système pour l'extraction de composants contenus dans un échantillon biologique sous forme liquide, lesdits composants étant aptes à se fixer sur des particules magnétiques, le système comprenant :
- une pipette (12) équipée d'au moins un cône de pipette tubulaire (20) comprenant une pointe (21) destinée au pipetage de liquide et d'un circuit d'aspiration et de refoulement dans chaque cône de pipette (20) ;
- au moins un support de puits (18a, 18b) ;
- un porte-pipette (14) comprenant :
∘ un socle (34) ;
∘ un évidement (56) formé dans le socle (34) et apte à loger de manière amovible chaque support de puits ;
∘ un support de pipette (36) comprenant un premier logement (38) dans lequel la pipette (12) est insérée, le premier logement (38) étant ouvert sur l'évidement (56) du socle (34), le support de pipette (36) étant mobile en translation par rapport au socle (34) selon une direction parallèle à un axe (X) des cônes de pipette (12) et mobile entre une première position dans laquelle la pointe (21) de chaque cône de pipette (36) est logée dans un puits (62, 69) du support de puits (18a, 18b) et au moins une deuxième position dans laquelle ladite pointe (21) est en dehors dudit puits (62, 69) ;
∘ un second logement (78) faisant face à chacun des cônes de pipette (20) dans une position au-dessus de la pointe (21) de celui-ci lorsque le support de pipette est dans la première position, et le second logement faisant face à la pointe du cône de pipette lorsque le support de pipette est dans la deuxième position ; et
- une pièce aimantée (16) logée de manière amovible dans le second logement (78).

23. Système selon la revendication 22, dans lequel le porte-pipette est conforme à l'une quelconque des revendications 15 à 21.

24. Support de puits (18b), comprenant une pièce (64) dans laquelle sont formés des évidements (66) pour la réception des puits (69), et au moins un aimant (68) faisant face à chacun des évidements (66) formés dans ladite pièce (64).

## Patentansprüche

1. Verfahren zur Entnahme von in einer biologischen Probe in flüssiger Form enthaltenen Bestandteilen, wobei die Bestandteile sich an magnetischen Partikeln fixieren können, wobei das Verfahren enthält:
- eine Phase (106) des Mischens der Probe mit den magnetischen Partikeln;
- eine Phase (110) des Ansaugens der Mischung von einem Schacht in einen rohrförmigen Pipettenkonus (20), der eine Spitze (21) enthält, die zur Flüssigkeitspipettierung bestimmt ist;
- eine Phase des Einfangens (112) der magnetischen Partikel auf einer Innenwand des Pipettenkonus (20):
∘ durch Anlegen eines ersten Magnetfelds (16) an den Pipettenkonus (20), wobei das Feld die magnetischen Partikel in einem vorbestimmten Bereich des Pipettenkonus (20), "Einfangbereich" genannt, oberhalb von dessen Spitze anziehen und halten kann;
∘ und durch Anwenden mindestens eines Ansaug- und Abgabezyklus der im Pipettenkönus (20) enthaltenen Mischung in einen Schacht (62);
- mindestens eine Phase (114, 116) des Waschens der auf der Innenwand des Pipettenkonus (20) eingefangenen Partikel:
∘ durch Abgabe der im Pipettenkonus (20) enthaltenen Mischung; und
∘ durch Anwenden ausgehend von einem eine Waschlösung enthaltenden Schacht (62) mindestens eines Ansaug- und Abgabezyklus der Waschlösung in den Pipettenkonus (20);
- eine Phase (118) des Wanderns der magnetischen Partikel auf der Innenwand des Pipettenkonus (20) vom Einfangbereich bis zur Spitze (21) des Pipettenkonus (20), indem eine relative Verschiebung des Pipettenkonus (20) bezüglich des ersten Magnetfelds (16) durchgeführt wird;
- und eine Phase (120) der Übertragung der magnetischen Partikel, die in die Spitze (21) des Pipettenkonus (20) gewandert sind, in einen eine Lösung enthaltenden Wiedergewinnungsschacht (69).

2. Verfahren nach Anspruch 1, wobei die Verschiebung des ersten Magnetfelds (16) darin besteht, den Pipettenkonus (20) parallel zu einer Längsachse (X) des Konus (20) zu verschieben, und das erste Magnetfeld (16) konstant zu halten, wobei die Längsachse (X) des Pipettenkonus bei der Verschiebung des Pipettenkonus (20) in gleichem Abstand zum ersten Magnetfeld (16) bleibt.

3. Verfahren nach Anspruch 1 oder 2, wobei die Übertragungsphase (120) enthält:
- die Anordnung der Spitze (21) des Pipettenkonus (20) im Wiedergewinnungsschacht (69);
- und das Anlegen eines zweiten Magnetfelds (68) vom Boden des Wiedergewinnungsschachts (69), um die in der Spitze (21) des Pipettenkonus (20) enthaltenen magnetischen Partikel in den Wiedergewinnungsschacht (69) wandern zu lassen.

4. Verfahren nach Anspruch 3, wobei das zweite Magnetfeld (68) von einem Magnet erzeugt wird, der teilweise oder ganz unter der Spitze des Pipettenkonus positioniert ist.

5. Verfahren nach Anspruch 3 oder 4, wobei das an den Pipettenkonus angelegte erste Magnetfeld bei der Anwendung des zweiten Magnetfelds deaktiviert ist.

6. Verfahren nach einem der Ansprüche 3 bis 5, wobei die Übertragungsphase (120) die Deaktivierung des ersten Magnetfelds (16) gefolgt vom Anwenden von Ansaug- und Abgabezyklen der Lösung vom Wiedergewinnungsschacht (69) in die Spitze (21) des Pipettenkonus aufweist, wobei die Anwendung enthält:
- eine erste Phase der Anwendung der Zyklen auf einer ersten Frequenz;
- gefolgt von einer zweiten Phase der Anwendung der Zyklen auf einer zweiten Frequenz niedriger als die erste Frequenz.

7. Verfahren nach einem der vorhergehenden Ansprüche, das vor der Einfangphase (112) eine Phase des Umrührens der im Pipettenkonus enthaltenen Mischung durch die Anwendung mindestens eines Ansaug- und Abgabezyklus der Mischung in den Pipettenkonus enthält.

8. Verfahren nach einem der vorhergehenden Ansprüche, das vor der Übertragungsphase (120) mindestens eine Waschphase der auf der Innenwand des Pipettenkonus eingefangenen Partikel enthält:
- indem das Magnetfeld deaktiviert wird;
- indem ausgehend von einem eine Waschlösung enthaltenden Schacht mindestens ein Ansaug- und Abgabezyklus der Waschlösung in den Pipettenkonus angewendet wird;
- indem eine zweite Einfangphase auf einer Innenwand des Pipettenkonus angewendet wird:
∘ indem das erste Magnetfeld an den Pipettenkonus angelegt wird;
∘ und indem mindestens ein Ansaug- und Abgabezyklus der im Pipettenkonus enthaltenen Mischung in den die Waschlösung enthaltenden Schacht angewendet wird.

9. Verfahren nach einem der vorhergehenden Ansprüche, das vor der Übertragungsphase (120) mindestens eine Waschphase der auf der Innenwand des Pipettenkonus eingefangenen Partikel enthält:
- indem die Waschlösung in den Pipettenkonus angesaugt wird;
- dann, indem das erste an die magnetischen Partikel angelegte Magnetfeld moduliert wird, um diese auf der Innenwand des Pipettenkonus einzufangen;
- dann, indem die Waschflüssigkeit aus dem Pipettenkonus abgegeben wird.

10. Verfahren nach Anspruch 9, wobei die Modulation des ersten Magnetfeldes durchgeführt wird:
- indem der Pipettenkonus parallel zu einer Längsachse des Konus verschoben wird, und indem das erste Magnetfeld konstant gehalten wird;
- und/oder indem zueinander beabstandete Magnete vor den eingefangenen Partikeln vorbeigeführt werden.

11. Verfahren nach einem der vorhergehenden Ansprüche, wobei das Volumen des Pipettenkonus mindestens zehnmal mal so groß ist wie das Volumen des Wiedergewinnungsschachts.

12. Verfahren nach einem der vorhergehenden Ansprüche, wobei das Volumen der Mischung mindestens dreimal so groß ist wie das Volumen des Pipettenkonus.

13. Verfahren nach einem der vorhergehenden Ansprüche, wobei die Bestandteile zu der Gruppe gehören, die von den Nucleinsäuren, den Mikroorganismen, den Proteinen und den Peptiden gebildet wird.

14. Pipettenhalter (14), der enthält:
- einen Sockel (34);
- eine Aussparung (56), die im Sockel (34) geformt und fähig ist, einen Schachtträger (18a, 18b) entfernbar aufzunehmen;
- einen Pipettenträger (36), der eine erste Aufnahme (38) enthält, in die eine Pipette (12) eingeführt werden kann, die mit mindestens einem rohrförmigen Pipettenkonus (20) ausgestattet ist, der eine zur Flüssigkeitspipettierung bestimmte Spitze (21) enthält, wobei die erste Aufnahme (38) sich zur Aussparung (56) des Sockels öffnet, wobei der Pipettenträger (36) bezüglich des Sockels (34) gemäß einer Richtung parallel zu einer Achse (X) der Pipettenkoni (20) translationsbeweglich und zwischen einer ersten Stellung, in der die Spitze (21) jedes Pipettenkonus in einem Schacht (62, 69) des Schachtträgers (18a, 18b) angeordnet ist, und mindestens einer zweiten Stellung beweglich ist, in der die Spitze (21) sich außerhalb des Schachts (62, 69) befindet;
- eine zweite Aufnahme (78), die ein magnetisiertes Bauteil (16) entfernbar aufnehmen kann, wobei die zweite Aufnahme (78) jedem der Pipettenkoni (20) in einer Stellung oberhalb von dessen Spitze gegenüberliegt, wenn der Pipettenträger (36) in der ersten Stellung ist, und die zweite Aufnahme (78) der Spitze des Pipettenkonus (20) gegenüberliegt, wenn der Pipettenträger (36) in der zweiten Stellung ist.

15. Pipettenhalter nach Anspruch 14, wobei die erste Aufnahme (38) eine Öffnung zur stirnseitigen Einführung und Entnahme der Pipette (12) in der ersten Aufnahme (38) enthält.

16. Pipettenhalter nach Anspruch 14 oder 15, wobei die zweite Aufnahme (78) im Sockel (36) ausgeführt ist.

17. Pipettenhalter nach einem der Ansprüche 14 bis 15, der eine dritte Aufnahme im Pipettenträger (36) enthält, in der das magnetisierte Bauteil (16) entfernbar aufgenommen werden kann, um jedem Pipettenkonus (20) in einer Stellung über der Spitze (21) des Konus gegenüberzuliegen, wenn der Pipettenträger (36) in der zweiten Stellung ist.

18. Pipettenhalter nach Anspruch 17, wobei die zweite und die dritte Aufnahme in Verbindung stehen, und wobei der Pipettenträger Einrichtungen enthält, die das magnetisierte Bauteil entfernbar in der dritten Aufnahme halten können.

19. Pipettenhalter nach einem der Ansprüche 14 bis 18, wobei:
- der Sockel (34) mindestens ein drehbewegliches Zahnrad (42) enthält;
- und der Pipettenträger eine Zahnstange (40) enthält, die mit dem Zahnrad (42) zusammenwirkt, um der Pipettenträger (36) bezüglich des Sockels (34) bei der Drehung des Zahnrads in Translationsrichtung zu bewegen.

20. Pipettenhalter nach Anspruch 19, der eine Verriegelungs- (und Entriegelungs-)vorrichtung des Pipettenträgers (82) mindestens in der ersten Stellung enthält.

21. Pipettenhalter nach einem der Ansprüche 19 oder 20, der mindestens einen fest mit dem Zahnrad verbundenen Griff (50) enthält, um dieses zu drehen, und der entfernbar an einem fest mit dem Zahnrad eines anderen Pipettenhalters verbundenen Griff (50) befestigt werden kann.

22. System zur Entnahme von in einer biologischen Probe in flüssiger Form enthaltenen Bestandteilen, wobei die Bestandteile sich an magnetischen Partikeln fixieren können, wobei das System enthält:
- eine Pipette (12), die mit mindestens einem rohrförmigen Pipettenkonus (20), der eine zur Flüssigkeitspipettierung bestimmte Spitze (21) enthält, und mit einem Ansaug- und Abgabekreislauf in jedem Pipettenkonus (20) ausgerüstet ist;
- mindestens einen Schachtträger (18a, 18b);
- einen Pipettenhalter (14), der enthält:
∘ einen Sockel (34);
∘ eine Aussparung (56), die im Sockel (34) geformt ist und jeden Schachtträger entfernbar aufnehmen kann;
∘ einen Pipettenträger (36), der eine erste Aufnahme (38) enthält, in die die Pipette (12) eingeführt wird, wobei die erste Aufnahme (38) zur Aussparung (56) des Sockels (34) hin offen ist, wobei der Pipettenträger (36) bezüglich des Sockels (34) gemäß einer Richtung parallel zu einer Achse (X) der Pipettenkoni (12) translationsbeweglich und zwischen einer ersten Stellung, in der die Spitze (21) jedes Pipettenkonus (36) in einem Schacht (62, 69) des Schachtträgers (18a, 18b) aufgenommen ist, und mindestens einer zweiten Stellung beweglich ist, in der die Spitze (21) sich außerhalb des Schachts (62, 69) befindet;
∘ eine zweite Aufnahme (78), die jedem der Pipettenkoni (20) in einer Stellung über dessen Spitze (21) gegenüberliegt, wenn der Pipettenträger in der ersten Stellung ist, und die zweite Aufnahme der Spitze des Pipettenkonus gegenüberliegt, wenn der Pipettenträger in der zweiten Stellung ist; und
- ein magnetisiertes Bauteil (16), das entfernbar in der zweiten Aufnahme (78) aufgenommen wird.

23. System nach Anspruch 22, wobei der Pipettenhalter einem der Ansprüche 15 bis 21 entspricht.

24. Schachtträger (18b), der ein Bauteil (64), in dem Aussparungen (66) für den Empfang der Schächte (69) geformt sind, und mindestens einen Magnet (68) enthält, der jeder der in dem Bauteil (64) geformten Aussparungen (66) gegenüberliegt.

## Claims

1. A method for extracting components contained in a biological sample in liquid form, said components being capable of binding to magnetic particles, the method comprising:
- a phase (106) of mixing the sample with the magnetic particles;
- a phase (110) of suctioning the mixture from a well in a tubular pipette cone (20) comprising a tip (21) intended for pipetting liquid;
- a phase of capturing (112) the magnetic particles on an internal wall of the pipette cone (20):
∘ by applying a first magnetic field (16) to the pipette cone (20), said field being capable of attracting and holding the magnetic particles in a predetermined zone of the pipette cone (20), termed "capture" zone, above the tip of said cone;
∘ and by applying at least one cycle of suction and discharge of the mixture contained in the pipette cone (20) in a well (62);
- at least one phase (114, 116) of washing the particles captured on the internal wall of the pipette cone (20) by:
∘ discharging the mixture contained in the pipette cone (20); and
∘ applying, from a well (62) containing a washing solution, at least one cycle of suction and discharge of the washing solution in the pipette cone (20);
- a phase (118) of migration of the magnetic particles on the internal wall of the pipette cone (20), from the capture zone to the tip (21) of the pipette cone (20), by carrying out a relative movement of the pipette cone (20) relative to the first magnetic field (16);
- and a phase (120) of transferring said magnetic particles having migrated into the tip (21) of the pipette cone (20) into a recovery well (69) containing a solution.

2. The method as claimed in claim 1, wherein the movement of the first magnetic field (16) consists in moving the pipette cone (20) parallel to a longitudinal axis (X) of said cone (20), and in keeping the first magnetic field constant (16), the longitudinal axis (X) of the pipette cone remaining at equal distance from the first magnetic field (16) during the movement of the pipette cone (20).

3. The method as claimed in claim 1 or 2, wherein the transferring phase (120) comprises:
- placing the tip (21) of the pipette cone (20) in the recovery well (69);
- and applying a second magnetic field (68) from the bottom of the recovery well (69) so as to cause the magnetic particles contained in the tip (21) of the pipette cone (20) to migrate into the recovery well (69).

4. The method as claimed in claim 3, wherein the second magnetic field (68) is produced by a magnet positioned partially or entirely under the tip of the pipette cone.

5. The method as claimed in claim 3 or 4, wherein the first magnetic field applied to the pipette cone is deactivated during the application of the second magnetic field.

6. The method as claimed in any one of claims 3 to 5, wherein the transferring phase (120) comprises the deactivation of the first magnetic field (16) followed by the application of cycles of suction and discharge of the solution of the recovery well (69) in the tip (21) of the pipette cone, said application comprising:
- a first phase of applying the cycles at a first frequency;
- followed by a second phase of applying the cycles at a second frequency, lower than the first frequency.

7. The method as claimed in any one of the preceding claims, comprising, prior to the capturing phase (112), a phase of stirring the mixture contained in the pipette cone by applying at least one cycle of suction and discharge of said mixture in the pipette cone.

8. The method as claimed in any one of the preceding claims, comprising, prior to the transferring phase (120), at least one phase of washing the particles captured on the internal wall of the pipette cone by:
a. deactivating the magnetic field;
b. releasing the captured particles by applying, from a well containing a washing solution, at least one cycle of suction and discharge of the washing solution in the pipette cone;
c. applying a second phase of capturing on an internal wall of the pipette cone:
∘ by applying the first magnetic field to the pipette cone,
∘ and by applying at least one cycle of suction and discharge of the mixture contained in the pipette cone in the well containing the washing solution.

9. The method as claimed in any one of the preceding claims, comprising, prior to the transferring phase (120), at least one phase of washing the particles captured on the internal wall of the pipette cone:
- by suctioning the washing solution in said pipette cone;
- then by modulating the first magnetic field applied to the magnetic particles in order to capture said particles on the internal wall of the pipette cone;
- then by discharging the pipette cone washing liquid.

10. The method as claimed in claim 9, wherein the modulation of the first magnetic field is carried out:
- by moving the pipette cone parallel to a longitudinal axis of said cone, and by keeping the first magnetic field constant;
- and/or by passing magnets spaced out from one another in front of the captured particles.

11. The method as claimed in any one of the preceding claims, wherein the volume of the pipette cone is at least ten times greater than the volume of the recovery well.

12. The method as claimed in any one of the preceding claims, wherein the volume of the mixture is at least three times greater than the volume of the pipette cone.

13. The method as claimed in any one of the preceding claims, wherein the components belong to the group formed by nucleic acids, microorganisms, proteins, and peptides.

14. A pipette holder (14) comprising:
- a base (34);
- a recess (56) made in the base (34), which can removably house a well support (18a, 18b);
- a pipette support (36) comprising a first housing (38) into which can be inserted a pipette (12) equipped with at least one tubular pipette cone (20) comprising a tip (21) intended for pipetting liquid, the first housing (38) opening out onto the recess (56) of the base, the pipette support (36) being translationally mobile relative to the base (34) in a direction parallel to an axis (X) of the pipette cones (20) and mobile between a first position in which the tip (21) of each pipette cone is inserted in a well (62, 69) of the well support (18a, 18b) and at least one second position in which said tip (21) is outside said well (62, 69);
- a second housing (78) which can removably house a magnetized part (16), the second housing (78) facing each of the pipette cones (20) in a position above the tip thereof when the pipette support (36) is in the first position, and the second housing (78) facing the tip of the pipette cone (20) when the pipette support (36) is in the second position.

15. The pipette holder as claimed in claim 14, wherein the first housing (38) comprises an opening for the frontal insertion of the pipette (12) into and the frontal removal of the pipette (12) from the first housing (38).

16. The pipette holder as claimed in claim 14 or 15, wherein the second housing (78) is made in the base (36).

17. The pipette holder as claimed in any one of claims 14 to 15, comprising a third housing in the pipette support (36) into which the magnetized part (16) can be removably inserted in order to face each pipette cone (20) at a position above the tip (21) of said cone when the pipette support (36) is in the second position.

18. The pipette holder as claimed in claim 17, wherein the second and third housings communicate, and wherein the pipette support comprises means capable of removably maintaining the magnetized part in the third housing.

19. The pipette holder as claimed in any one of claims 14 to 18, wherein:
- the base (34) comprises at least one toothed wheel (42) which can rotate;
- and the pipette support comprises a rack (40) which engages with the toothed wheel (42) in order to translationally move the pipette support (36) relative to the base (34) during the rotation of the toothed wheel.

20. The pipette holder as claimed in claim 19, comprising a device for locking and unlocking the pipette support (82) at least in the first position.

21. The pipette holder as claimed in either of claims 19 or 20, comprising at least one handle (50) rigidly connected to the toothed wheel for turning said wheel and capable of removably attaching to a handle (50) rigidly connected to the toothed wheel of another pipette holder.

22. A system for extracting components contained in a biological sample in liquid form, said components being capable of binding to magnetic particles, the system comprising:
- a pipette (12) equipped with at least one tubular pipette cone (20) comprising a tip (21) intended for pipetting liquid and with a circuit for suction and discharge in each pipette cone (20);
- at least one well support (18a, 18b);
- a pipette holder (14) comprising:
∘ a base (34);
∘ a recess (56) made in the base (34), which can removably house each well support;
∘ a pipette support (36) comprising a first housing (38) into which the pipette (12) is inserted, the first housing (38) opening out onto the recess (56) of the base (34), the pipette support (36) being translationally mobile relative to the base (34) in a direction parallel to an axis (X) of the pipette cones (12) and mobile between a first position in which the tip (21) of each pipette cone (36) is inserted in a well (62, 69) of the well support (18a, 18b) and at least one second position in which said tip (21) is outside said well (62, 69);
∘ a second housing (78) facing each of the pipette cones (20) in a position above the tip (21) thereof when the pipette support is in the first position, and the second housing facing the tip of the pipette cone when the pipette support is in the second position; and
- a magnetized part (16) removably inserted in the second housing (78).

23. The system as claimed in claim 22, wherein the pipette holder is in accordance with any one of claims 15 to 21.

24. A well support (18b), comprising a part (64) in which recesses (66) for receiving the wells (69) are made, and at least one magnet (68) facing each of the recesses (66) made in said part (64).
